# EUROPEAN PATENT APPLICATION

(11) **EP 1 676 859 A1**
(43) Date of publication of application: **05.07.2006**
(21) Application number: 04031023.7
(22) Date of filing: 30.12.2004
(51) Int. Cl.: C07K 14/47, G01N 33/68, C07K 7/64

(54) **Immunogenic compositions of cyclic peptides derived from the beta-amyloid peptide**

(71) Applicant: Pevion Biotech Ltd., 3018 Bern (CH)
(72) Inventor: Zurbriggen, Rinaldo, 3185 Schmitten (CH)
(74) Representative: Viering, Jentschura & Partner

(57) **Abstract**

The present invention relates to methods and compositions for the elicitation of an immune response, in particular against amyloid beta-peptides, in particular by the combinatory use of virosomes as adjuvants and a synthetic amyloid beta-peptide antigen.

## Description

### Field of the invention

The present invention relates to methods and compositions for the elicitation of an immune response, in particular against amyloid beta-peptides, in particular by the combinatory use of virosomes as adjuvants and a synthetic amyloid beta-peptide antigen.

### Background of the invention

The generic term amyloid refers to a group of proteinaceous deposits, sharing common morphological properties and staining behaviours. Consequently, amyloidosis refers to pathologic accumulation of amyloid fibres. One of the best-known disorders involving the accumulation of protein aggregates, is Alzheimer's Disease (AD). AD is a progressive degenerative neuronal disorder of insidious onset responsible for cognitive decline and dementia in millions of patients associated with loss of neurons and the appearance of reactive glia. AD proceeds in stages, gradually destroying memory, reason, judgement, behaviour, personality, language and cognitive abilities. Said impairments relate to the loss of neurons and the signalling between them. AD strikes approximately 20 million people worldwide and can affect persons as young as 40-50 years of age, but the large majority is of older age, with estimates of the affected population reaching as high as nearly 50% by the age of 85-90. The exact time of onset is unknown, as the presence of the disease is, in the early stages, difficult to determine without the high risk of a brain biopsy, but early onset AD has been traced to genetic origins. Considering the necessary provision of health and ancillary care for the AD patients, the required expenses are enormous.

The neuropathological hallmark of this disease is the abundant presence of extracellular proteinaceous plaques in the brain regions related to memory and cognition of the affected individuals. This insoluble neuritic protein deposits, termed amyloid plaques, are complex aggregations formed from a peptide cleaved from a larger precursor protein, whose amino acid sequence predisposes it to aggregation. The frequency and distribution of these plaques correlates with the severity of the disease. Although a number of other neurological impairments are associated with proteinaceous plaques, Alzheimer's Disease is the most common and best characterized.

The major component of amyloid plaques is a 42 amino acid long polypeptide, the β-amyloid peptide (Aβ), that can exist in two distinct conformational states: a mostly α-helical coiled and benign soluble form and a mainly β-sheet form that aggregates spontaneously into insoluble deposits. The peptide is derived by proteolysis from a large transmembrane glycoprotein protein with neurotrophic functions, called amyloid precursor protein (APP), ubiquitously expressed from a gene located on chromosome 21 in all tissues and predominantly in the central nervous system. Proteolytic cleavage of this precursor by secretases follows two main routes. The major route employs α-secretase that cleaves APP near its center into two large products, termed APPsα and C83. Subsequently, C83 is further hydrolysed by γ-secretase, yielding a small peptide known as p3. The second, minor APP-processing pathway is based on the N- and C-terminal cleavage of APP by β- and γ-secretase and leads to the β-amyloid peptide, whose aggregates are toxic to neurons and are implicated in the neurodegeneration that underlies the decline of cognitive functions. A preponderance of evidence indicates that a chronic imbalance in the production and clearance of Aβ protein initiates pathologic responses, including neuritic and synaptic abnormalities, neurofibrillary tangles and loss of neurotransmitters. Although it is widely accepted that the peptides found in these plaques damage the nervous system, it is currently not completely understood by which mechanism the pathology is actually driven. One pathway for Aβ-induced neuronal damage may involve inflammatory cells, especially reactive microglia found to be associated with neuritic and core plaques. Histological studies have shown that nearly all amyloid plaques in the brain of affected individuals are surrounded by clusters of reactive microglia. These cells release such bioactive agents as proteases, cytokines, free radicals, nitric oxide and neurotoxic amines, thus evoking neuron-killing and therefore are likely to significantly contribute to the immunopathology of diseases associated with amyloid plaques. In vitro studies have confirmed the assumption that quiescent microglia can be activated through the contact with isolated amyloid plaque fragments and in response produce a neurotoxin that was found to destroy hippocampal pyramidal cells. These findings support the idea that the immune response is an important part of the pathology of AD.

The discovery of point mutations in the amyloid precursor protein in some rare families with an autosomal dominant form of AD provided evidence that abnormalities in the beta-amyloid metabolism are involved in the development of AD. These mutations occur in direct proximity to the N- and C-terminal cleavage points necessary for the generation of Aβ peptide from its precursor protein and thereby affect the proteolytic cleavage by cellular secretases. However, more than 99% of the AD patients suffer from the non-familial form, in which amyloidosis is explained by a decreased clearance of Aβ or an increase in its aggregation properties.

Although 20 years have passed since the initial report of the purification and characterization of Aβ derived from the brains of patients with AD, no therapy has been approved, which specifically targets this protein. Several approaches, currently in development, in order to slow, stop or reverse the progression of AD, include anti-inflammatory drugs, metal complexing agents, secretase inhibitors, neurotrophic agents and even vitamins. The three main approaches aim at the prevention of the plaque formation and the clearance of already formed amyloid deposits. One principle targets the secretases and tries to modulate their function in a way to promote α-secretase activity and at the same time blocking β-and γ-secretase. As their biological properties remain ambiguous and their function is still not completely understood, this approach is quite uncertain. A second focus lies on the inhibition of Aβ fibril formation or the reversal of aggregation that has already taken place, by using specific inhibitors. The third and most promising strategy for the prevention and/or treatment of AD is the immunization against Aβ.

As an abnormal substance of an endogenous peptide, the amyloid plaques present the immune system with severe difficulties. Normally the Aβ elicits a very low humoral immune response, i.e. its immunogenicity is very weak, but the aggregation of monomeric peptides to fibrils creates a neo-epitope, against which antibodies can be generated. To eliminate the amyloid, the system has to raise antibodies against the neo-epitopes in the aggregated proteins without provoking an autoimmune response, which could destroy the amyloid peptide- or its precursor-producing tissue. In addition the Aβ forms its poorly soluble deposits in a location, the brain, which is to a large extent isolated from the system of acquired immunity. This limits the capacity of the plaques to generate or react with the humoral immune system. Therefore, the development of an effective vaccine against Aβ was considered as one of the most promising principles in recent research for the treatment or prevention of AD. Various research groups have reported on the benefits of immunization of different lines of APP transgenic mice with AB or derivatives thereof. Using this approach the development of amyloid plaques could be prevented, existing plaques were removed, the deposition of fibrillar Aβ was significantly reduced, and the cognitive dysfunction could be alleviated. Even passive immunization proved successful to reduce pathology of the amyloid plaques and reverse memory impairment.

Despite these promising concepts concerning immunization as a solution, to date all these efforts have been proven to be ineffective, as initial studies with humans were not successful. Inoculation of humans with synthetic Aβ₁₋₄₂ had the severe side effect of cerebral inflammation in some patients in phase II clinical trials, the cause of this adverse side effect being most likely Aβ₁₋₄₂ toxicity and/or autoimmunity. Given the serious side effects of Aβ₁₋₄₂ vaccination, use of non-toxic immunogenic Aβ derivatives is assumed to be a safe alternative, considering that the main immunogenic epitopes are contained in the N-terminal part of the amyloid peptide.

First promising findings indicated that immunization with non-amyloidogenic Aβ derivatives might be a safer therapeutic approach. In these experiments antibodies implicated to peripherally reduce the systemic amount of soluble Aβ played a pivotal role, as they provide a method to circumvent the potentially detrimental side effects arising from intracerebral immune reactions.

Additionally, diagnosis of AD is also burdened with several difficulties. By definition, AD is only definitively diagnosed through the examination of brain tissue, usually at autopsy. The currently recommended minimum microscopic criteria for AD diagnosis is based on the number of neuritic plaques composed of Aβ found in the brain. The Aβ peptide can easily be identified by staining brain sections with thioflavin S or Congo Red. Congo red-stained amyloid is characterized by a dichromatic appearance, exhibiting a yellow-green polarization color as a result of the beta-pleated sheet structure of the amyloid proteins. Genetic tests for the diagnosis of AD rely on suspected risk factors, as Apolipoprotein E epsilon4 (55% of the ApoE ε4/ε4 homozygotes develop AD by age 80), the secretases Presenilin 1 and 2 or the APP itself, but have proved inappropriate for general diagnostic means. Therefore, great need exists for compositions and methods allowing accurate diagnosis of AD and other neurodegenerative diseases due to beta amyloid deposition without being based on brain biopsy or autopsy.

Thus, what is needed are compositions and methods for the diagnosis, treatment and/or the prevention of the formation of beta amyloid plaques, without the risk of detrimental side effects.

### Summary of the invention

The present invention provides cyclic Aβ-derived peptides characterized in that they provoke or enhance an immune response against amyloid peptides in an organism, said organism being a human or animal. More specifically, the present invention features synthetic immunogenic peptide antigens directed to the diagnosis and treatment of amyloid-associated diseases, e.g. Alzheimer's Disease, but most importantly the vaccination against said disorders.

The invented compositions can further comprise additional adjuvants and/or delivery vehicles, to provoke the production of specific antibodies, thus stimulating the humoral immune system.

The invention targets not primarily the proteinaceous deposits in the brain, as this may be coupled to detrimental side effects arising from intracerebral immune reactions, but the soluble Aβ, not only present in the brain, but also cycling in the peripheral vessel system, thereby reducing the chance of accumulation of this molecule and, thus, hindering the formation of amyloid plaques.

The present invention provides cyclic peptides sharing significant sequence homology with Aβ for vaccination against amyloid-associated diseases. The conformational constrained structure of the invented cyclic peptides, allows overcoming the counterproductive flexibility and low metabolic stability of linear peptides.

Particularly, the antigenic peptides of the present invention are represented by the following formula: 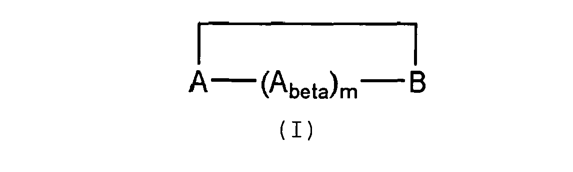 wherein A_{beta} is a peptide of 8-26 amino acid residue length derived from the β-amyloid peptide (Aβ₁₋₂₆; SEQ ID NO: 2) or an analog thereof containing a conservative amino acid substitution;
wherein m is 1 to 6;
wherein A and B represent a direct bond, a linking template or an amino acid sequence comprising 1 to 20 amino acids or derivatives thereof and are selected independently from each other; and
wherein A and B are covalently linked to cyclize the peptide.

In a highly preferred embodiment A_{beta} is a peptide of 12-20 amino acid residue length derived from the β-amyloid peptide (Aβ₁₋₂₆; SEQ ID NO: 2), preferably Aβ₁₋₁₆ (SEQ ID NO: 3). In another highly preferred embodiment A_{beta} is Aβ₁₁₋₂₆ (SEQ ID NO: 4).

In one preferred embodiment m is 1, 2 or 3, more preferably 1.

The antigenic peptides of the present invention have preferably a length of 10 to 196 amino acids.

In a certain embodiment, the peptides of the present invention have a length from 10 to 40 amino acid residues, preferably from about 12 to 30 amino acid residues, and more preferably from 14 to 20 amino acid residues.

If A and B represent a direct bond, any two of the amino acids of A_{beta}, preferably the N- and C-terminal amino acid, are covalently linked to cyclize the peptide. The covalent bond can be formed utilizing the N-terminal amino- and the c-terminal carboxy-group, but can also be formed by utilizing any side chain of any of the amino acids of A_{beta}.

If A and B represent a linking template, A and B together form a molecule or chemical group that cyclizes A_{beta}. The linking template fixes the Abeta chain in a favorable loop structure and, thus, constrains the flexibility of the antigenic peptide. In a preferred embodiment of the present invention the linking template is a C₅-C₁₄ mono- or polycyclic ring or ring system that optionally contains one or more heteroatoms selected from the group consisting of N, O and S. In a preferred embodiment said ring system may be substituted with at least two functional groups that allow the covalent bonding of the amino acid chain and are preferably selected from the group consisting of carboxy, hydroxy, amid, amino or imino. In one embodiment said imino group can also be part of said ring or ring system. Said linking template forms, preferably via said functional groups, covalent bonds with the N-terminal amino on one end and on the other end the C-terminal carboxy group of the amino acid chain of A_{beta}. In another embodiment those substituents covalently bond side chain groups of any of the amino acids of A_{beta}. The C₅-C₁₄ mono- or polycyclic ring or ring system can be further substituted with C₁-C₆ alkyl, C₁-C₆ alkoxy, aryl, aryl-C₁-C₆ alkyl, aroyl-C₁-C₆ alkyl, allyl, halogen, NO₂ or a group of formula CH₂-COOR', wherein R' is hydrogen, C₁-C₆ alkyl, substituted C₁-C₆ alkyl, aryl-C₁-C₆ alkyl, aryl, aroyl-C₁-C₆ alkyl or allyl. If present, the latter groups are preferably substituted by at least two of the above explained functional groups so that said linking forms, via said functional groups, covalent bonds with an amino group and a carboxy group of A_{beta}.

In a highly preferred embodiment the linking template is selected form the group comprising compounds of the following formulae, whereby the covalent bonds to A_{beta} are formed via the (bold) carboxy and amino/imino moiety: 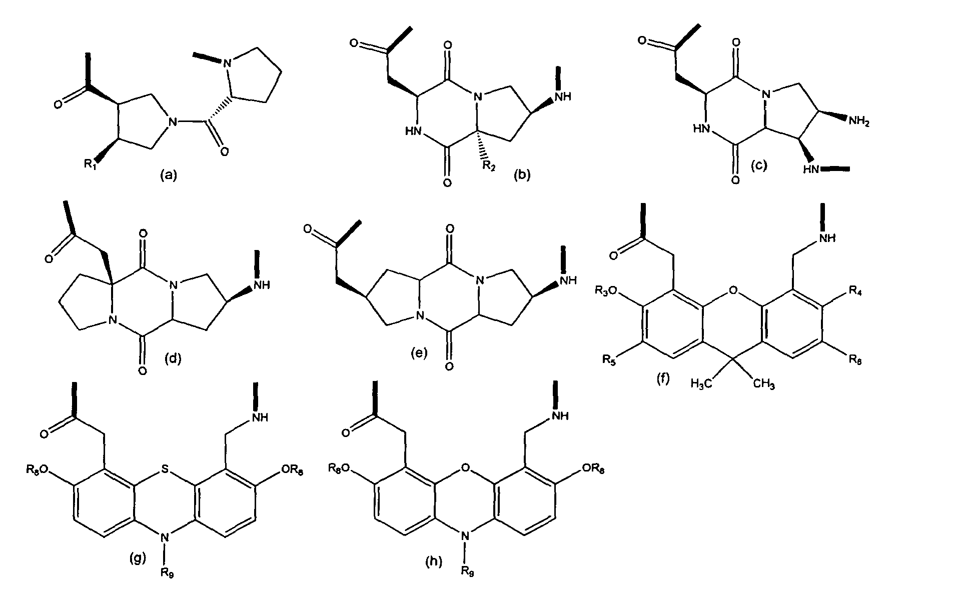 wherein R₁ is hydrogen or an amino group;
R₂ is hydrogen or a group of formula CH₂-COOR₉;
R₃ is hydrogen, C₁-C₆ alkyl or aryl-C₁-C₆ alkyl;
R₄ is hydrogen, C₁-C₆ alkyl, C₁-C₆ alkoxy or aryl-C₁-C₆ alkyl;
R₅ is hydrogen, C₁-C₆ alkyl, substituted C₁-C₆ alkyl, aryl, Br or NO₂;
R₆ is hydrogen, C₁-C₆ alkyl, substituted C₁-C₆ alkyl, aryl, Br or NO₂;
R₇ is hydrogen, C₁-C₆ alkyl, substituted C₁-C₆ alkyl, or aryl-C₁-C₆ alkyl;
R₈ is hydrogen, C₁-C₆ alkyl, substituted C₁-C₆ alkyl, or aryl-C₁-C₆ alkyl; and
R₉ is hydrogen, C₁-C₆ alkyl, substituted C₁-C₆ alkyl, aryl-C₁-C₆ alkyl, aryl, aroyl-C₁-C₆ alkyl or allyl.

If A and B are amino acid residues, then they can be any naturally occurring amino acid or any non-naturally occurring amino acid or derivatives thereof. Non-naturally occurring amino acids include, but are not limited to, hydroxyprolin, aminoprolin, thyroxin, ornithine, norvaline, norleucine, beta-alanine, gamma-amino butyric acid, homoserine, citrulline and the like. Naturally occurring amino acids include glycine, alanine, valine, leucine, isoleucine, methionine, proline, phenylalanine, tyrosine, tryptophane, serine, threonine, cysteine, glutamine, asparagine, histidine, lysine, arginine, glutamic acid and aspartic acid. Also included are explicitly both enantiomers of above-listed amino acids, i.e. the L- and the respective D-form.

In one preferred embodiment, A is 4-aminoprolin and B is D-Prolin.

The cyclization can be achieved via direct coupling of the N- and C-terminus to form a peptide bond, but can also occur via the amino acid side chains. Furthermore it can be based on the use of other functional groups, including but not limited to amino, hydroxy, sulfhydryl, halogen, sulfonyl, carboxy or thiocarboxy. These groups can be located at the amino acid side chains or be attached to their N- or C-terminus.

In a highly preferred embodiment the cyclic peptide of formula I is Cyclo(cis-4-Amino-Pro-Asp-Ala-Glu-Phe-Arg-His-Asp-Ser-Gly-Tyr-Glu-Val-His-His-Gln-Lys-D-Pro).

The amino acids of A and B may be independently from each other modified with lipid moieties. Said lipid moiety is a mostly hydrophobic molecule composed of one or more hydrophobic groups including but not limited to Lipid A, cholesterol, sphingolipids, glycerolbased lipids, diacylglycerol, PEG-lipids, dietherlipids, 1,2-Dioleoyloxy-3-trimethylammonium-propane (DOTAB), 1,2-Dioleoyloxy-3-dimethylammonium-propane (DODAPCl), N,N-Dioleoyl-N, N-dimethylammonium(DODAC), fatty acids, isoprenoids, sphingosin and derivatives thereof. The fatty acids include, but are not limited to caproic acid, caprylic acid, capric acid, lauric acid, myristic acid, palmitic acid, stearic acid, arachidic acid, behenic acid, lignoceric acid, myristoleic acid, palmitoleic acid, petroselinic acid, elaidic acid, , oleic acid, linoleic acid, linolelaidic acid, cis-vaccenic acid, linolenic acid, arachidonic acid, cis-11-eicosenoic acid, erucic acid, cis-4,7,10,13,16,19-Docosahexaenoic acid and nervonic acid. The glycerolbased lipids include, but are not limited to phosphatidylethanolamine, phosphatidylserine, phosphatidylcholin (lecithin), phosphatidylinositol, phosphatidylglycerol, cardiolipin, plasmalogen, phosphalic acid and lysophosphatidic acid. The isoprenoids include, but are not limited to farnesyl and geranylgeranyl.

The lipid moiety can be attached directly or via a linker to either or both of the amino acids of A and/or B. Direct linkage is achieved via an amid, ether, thioether, ester or thioester bond. The linker can be any compound, but preferably is chosen from the group of dicarboxylic acids, having 3 to 10 carbon atoms, and most preferably is succinic acid. The modification can take place at the side chain, or the respective amino or carboxy group of A or B. The lipid moiety can be any phospholipid, preferably phosphatidylethanolamine. It can be optionally coupled to a linker, such as succinic acid. In a one preferred embodiment the lipid moiety is diacyl-glycero-phosphoethanolamino-succinate, and more preferably 1,3-dipalmitoyl-glycero-2-phosphoethanolamino-succinate.

In the most preferred embodiment the antigenic peptide is Cyclo(cis-4-Amino-Pro(succinyl-(1,3-dipalmitoyl-glycero-2-phosphoethanolamino))-Asp-Ala-Glu-Phe-Arg-His-Asp-Ser-Gly-Tyr-Glu-Val-His-His-Gln-Lys-D-Pro).

The invention also relates to compositions comprising at least one antigenic peptide represented by formula I as defined above.

The peptide antigens may be coupled to or incorporated into suitable delivery vehicle, such as a microparticles including microspheres and nanospheres, polymeres, bacterial ghosts, bacterial polysaccharides, attenuated bacterias, virus like particles, attenuated viruses, ISCOMS, , liposome or virosome (IRIV). Specifically the lipid modified peptide antigens can, thus, be anchored on the surface of lipid containing delivery vehicles, such as liposomes and virosomes.

The use of virosomes and/or liposomes as antigen delivery vehicles for the above-mentioned compounds and inoculating a subject therewith is also part of the current invention. The relatively low immunogenicity of the amyloid peptide antigen can be increased by several orders of magnitude by the combination of antigen and virosome, inducing the production of measurable titers of highly specific antibodies. These compositions are highly suitable for the diagnosis, treatment or prevention of amyloid-associated disorders.

The method of preparing the described antigen/virosome composition comprises the modification of the disclosed peptides with a lipid moiety, allowing attachment to the virosome membrane surface. Alternatively the antigenic peptides can be transported unmodified inside the virosome.

In one preferred embodiment of the invention the antigen delivery vehicle is a virosome and the antigenic peptide is attached to the virosomal surface via a lipid moiety.

The present invention also provides the use of the antigenic peptides represented by formula I and the immunogenic compositions comprising said peptides for preparation of a pharmaceutical composition for the diagnosis and treatment of amyloid-associated diseases as well as the use of said peptides and compositions for the preparation of a pharmaceutical composition for the vaccination of a subject against said disorders. In one embodiment of this invention a combination of a beta amyloid antigen and a virosome is used to facilitate an increase in the level of antibodies specific for amyloid beta peptide, helpful in diagnosis, treatment or prevention of said disorder.

The immunogenic compositions of the present invention also comprise the combination of the invented peptide antigens with adjuvants, known to those skilled in the art, comprising but not limited to short nucleic acid stretches, peptides or protein fragments and derivatives thereof, aluminium salts and oligo- or polysaccharides. All these adjuvants can be used alone or in combination, creating an adjuvant system. As they can further stimulate immune response, their use in addition to or instead of virosomes is part of the invention.

The present invention also comprises methods for the preparation of the peptides of formula I, comprising the steps of sequentially synthesizing the peptide and cyclizing the peptide. The peptide antigens of the present invention can be prepared in a wide variety of ways, known to those skilled in the art. Because of its relatively small size, the peptide can be synthesized in solution or, more sophisticated, on a solid phase, for example a synthetic resin. Various automatic synthesizers are commercially available to date and can be used in accordance with well-investigated synthesis protocols. Alternatively, the peptides can be expressed recombinantly from synthetic genes in appropriate organisms. These techniques are also readily available for those skilled in the art. The methods for preparing the peptide of formula I can optionally further comprise the step of modifying the peptide with a lipid moiety as described above.

The invention also relates to the peptides obtainable by above-described methods, being represented by the following formula: 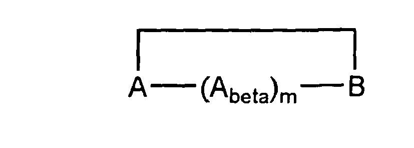 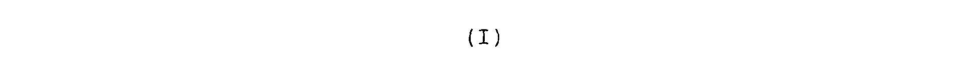 wherein A_{beta} is a peptide of 8-26 amino acid residue length derived from the β-amyloid peptide (Aβ₁₋₂₆; SEQ ID NO: 2) or an analog thereof containing a conservative amino acid substitution;
wherein m is 1 to 6;
wherein A and B represent a direct bond, a linking template or are selected independently from each other and stand for an amino acid sequence comprising 1 to 20 amino acids or derivatives thereof; and
wherein A and B are covalently linked to cyclize the peptide.

The present invention also contemplates antibodies or antigen binding fragments thereof, which bind to the invented compounds and inhibit amyloid deposition and fibril formation. Included in the invention are different types of antibodies, e.g. monoclonal, polyclonal or bispecific antibodies and epitope-recognizing fragments thereof. Furthermore, the antibodies may be chimeric antibodies, comprising parts of a human antibody as well as parts of antibody regions from other species.

The antibodies of this invention preferably recognize human Abeta proteins or peptides, but also include antibodies directed against amyloid peptides from other species, preferably mammals.

The invention further relates to a method for the preparation of an antibody that specifically recognizes said cyclic peptides, comprising the following steps:
(a) immunizing an organism with an immunogenic composition comprising at least one of the antigenic peptides of formula I;
(b) isolating the antibodies generated by the inoculation in step (a); and
(c) screening the antibodies obtained in step (b) for their specific recognition of the Aβ peptide fragments or variants thereof.

The invention also relates to an antibody obtainable by the above-described method and the use of said antibody for the preparation of a pharmaceutical for the diagnosis, treatment or prevention of an amyloid-associated disease.

### Brief description of figures

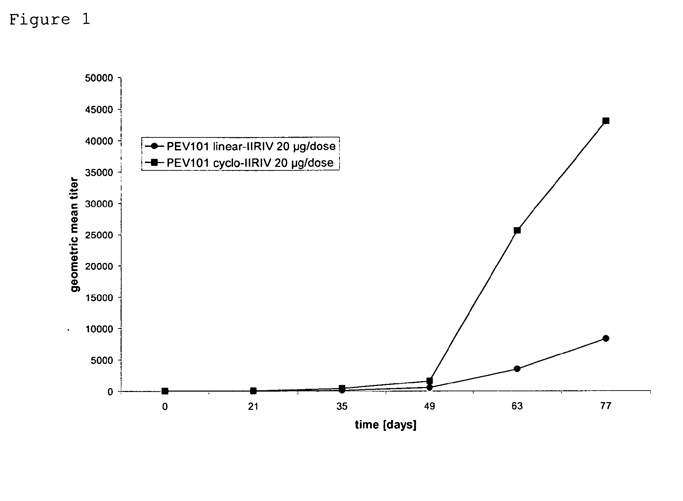

**Figure 1** shows the mean antibody titers of mice immunized with 20 µg doses of (Cyclo(cis-4-Amino-Pro(succinyl-(1,3-dipalmitoyl-glycero-2-phosphoethanolamino))-Asp-Ala-Glu-Phe-Arg-His-Asp-Ser-Gly-Tyr-Glu-Val-His-His-Gln-Lys-D-Pro) coupled to IRIVs and mice immunized with the linear counterpart of the immunogenic peptide coupled to IRIVs over a time course of 77 days after immunization.

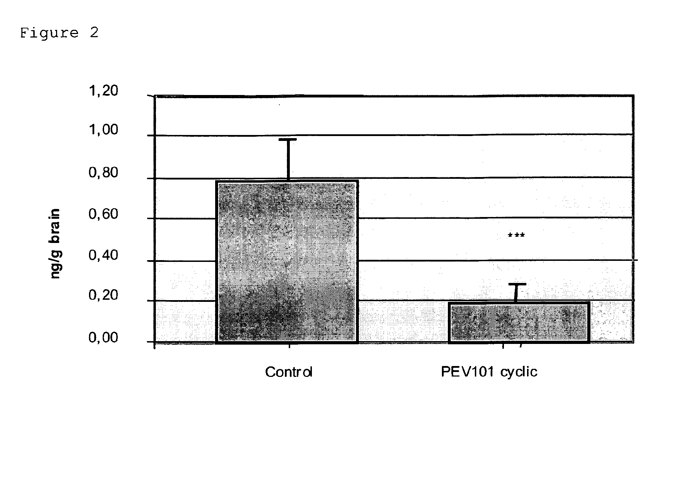

**Figure 2** shows the concentrations of soluble Aβ in mice immunized with (Cyclo(cis-4-Amino-Pro(succinyl-(1,3-dipalmitoyl-glycero-2-phosphoethanolamino))-Asp-Ala-Glu-Phe-Arg-His-Asp-Ser-Gly-Tyr-Glu-Val-His-His-Gln-Lys-D-Pro) in comparison to a untreated control group.

### Detailed description of the invention

### Synthetic peptide antigens and vaccines

The present invention provides peptides derived from Aβ that are effective in eliciting or increasing an immune response in a human or animal against amyloid peptides and, thus, is directed to compositions and methods for the diagnosis, treatment and prevention of amyloid-associated diseases. The terms "Aβ", "β-amyloid peptide" "amyloid peptides" or "Abeta" are used interchangeably throughout the present invention and refer to a peptide of 39-43 amino acids (Aβ₁₋₃₉, Aβ₁₋₄₀, Aβ₁₋₄₁, Aβ₁₋₄₂ or Aβ₁₋₄₃), being the main component of the characteristic amyloid plaques in Alzheimer's Disease. Aβ is generated by processing of the much larger transmembrane protein APP by two cellular proteases, β- and γ-secretase. The peptide predominantly found in the plaques is the Aβ₁₋₄₂ (SEQ ID NO: 1). The shorter variants Aβ₁₋₃₉, Aβ₁₋₄₀ and Aβ₁₋₄₁ differ from Aβ₁₋₄₂ by the omission of 1-3 amino acids from the C-terminal end. Aβ₁₋₄₃ has an additional threonine at the C-terminus. Soluble Aβ refers to a form of the peptide, which is not yet aggregated. In this context the term "prevention" refers to vaccination and immunization against said diseases. "Vaccination" refers to the process of inoculating an organism with an antigen to elicit an immune response, that helps to prevent or treat the disease or disorder the antigen is connected with in the future. The term "immunization" is interchangeable with vaccination. Passive immunization refers to the administration of antigen specific antibodies, not produced by the organism to be treated. The term "peptide" refers to a small number of amino acids linked together by a peptide bond. To more clearly specify peptides, prefixes such as di-, tri-, oligo- or poly- are used. The "antigens" of the present invention are short peptide stretches of 8-26 amino acids length that share significant sequence homology with the β-amyloid peptide Aβ₁₋₂₆ (SEQ ID NO: 2).

A variant of a peptide of the present invention involves an amino acid substitution, conservative amino acid substitutions typically will be preferred, i.e., substitutions which retain a property of the original amino acid such as size, charge, hydrophobicity, conformation, etc. Examples of conservative substitutions of amino acids include substitutions made among amino acids within the following groups: (Amit, A. G., *et al*. 1986, Science 233:747-753) M, I, L, V; (Anders, R. F., *et al.* 1998, Vaccine 16:240-247) F, Y, W; (Barlow, D. J., *et al.* 1986, Nature 322:747-748) K, R, H; (Cheng, Q. and A. Saul. 1994, Mol. Biochem. Parasitol. 65:183-187) A, G; (Cohen, S., *et al.* 1961, Nature 192:733-737) S, T; (Coley, A. M., *et al.* 2001, Protein Eng 14:691-698) Q, N; and (Collins, W. E., *et al.* 1994, Am. J. Trop. Med. Hyg. 51:711-719) E, D. Other suitable substitutions are easily established by the person of skill and may additionally be determined by reference to publications such as Voet, *Biochemistry,* Wiley, 1990; Stryer *Biochemistry* 4^{th} Ed., Freeman New York, 1995 ; *Peptide Chemistry. A Practical Textbook,* 2nd ed., Miklos Bodanszky, Springer-Verlag, Berlin, 1993; *Principles of Peptide Synthesis,* 2nd ed., Miklos Bodanszky, Springer-Verlag, Berlin, 1993; *Chemical Approaches to the Synthesis of Peptides and Proteins,* P. Lloyd-Williams, F. Albericio, E. Giralt, CRC Press, Boca Raton, 1997; *Bioorganic Chemistry: Peptides and Proteins,* S. M. Hecht, Ed., Oxford Press, Oxford, 1998, *Synthetic Peptides: A User's* Guide, Gregory A. Grant (Editor), Oxford University Press, 2002, and the like.

In particular, the invention is directed to compositions comprising above-mentioned peptides for eliciting anti-amyloid antibodies. These compositions can comprise other ingredients such as adjuvants and/or delivery vehicles to further potentiate immune response. "Adjuvant" refers to a substance that is capable of potentiating the immunogenicity of an antigen. Adjuvants can be one substance or a mixture of substances and function by acting directly on the immune system or by providing a slow release of an antigen. Examples of adjuvants are aluminium salts, polyanions, bacterial glycopeptides and slow release agents as Freund's incomplete. "Delivery vehicle" refers to a composition that helps to target the antigen to specific cells and to facilitate the effective recognition of an antigen by the immune system. The best-known delivery vehicles are liposomes, virosomes, microparticles including microspheres and nanospheres, polymeres, bacterial ghosts, bacterial polysaccharides, attenuated bacterias, virus like particles, attenuated viruses and ISCOMS.

More specifically, the invention provides compositions and methods to enhance the humoral immune response against Aβ, especially by the production of specific antibodies that can be used to prevent or delay the neuropathology linked to amyloid-associated diseases. "Humoral immunity" or "humoral immune response" both refer to B-cell mediated immunity and are mediated by highly specific antibodies, produced and secreted by B-lymphocytes (B-cells). Secreted antibodies bind to antigens and facilitate neutralization, opsonization, and activation of the complement system and destruction of pathogens flagged by this means.

Such compositions are not only useful for the vaccination, but also for the treatment and the diagnosis of diseases associated with amyloid protein abnormalities, e.g. Alzheimer's Disease and related disorders. Aim and principle of the invention is the peripheral reduction of systemic, soluble Aβ by antibodies directed against this peptide, thereby, on the one hand preventing the accumulation of this molecule otherwise leading to aggregation and on the other hand circumventing the potentially detrimental side effects arising from intracerebral immune reactions.

To elicit an immune response, antigens have to be taken up and processed by a special type of cell, the so-called antigen-presenting cells (APCs). The three cell types able to present antigens, are dendritic cells, macrophages and B-lymphocytes. The uptake is facilitated by phago- or endocytosis and the processing is done in vesicles or the cytosol. Key players in the presentation of the antigen on the cell surface are a class of proteins termed major histocompatibility complex proteins (MHC). These molecules are encoded by the most polymorphous gene family in the human genome located on chromosome 6 and can be subdivided into class I and II. The topology of both classes of molecules is such that they can bind and present as broad a spectrum of peptides 8 to 16 amino acids in length as possible. Thus, a very effective immunosurveillance is ensured. The copy number of a defined antigenic peptide on the surface of an antigen-presenting cell is quite low (about one hundred), given the total number of about ten thousand peptide receptors. But this feature accounts for a very heterogeneous mixture of antigenic peptides on the surface of each APC.

MHC class I molecules bind and present samples of cellular peptides, including endogenous as well as translated and processed viral or tumor antigens and activate the cellular, cytotoxic immune response via CD8+ (cytotoxic T-cells) cells. Autoimmunity against endogenous molecules is normally prevented by the positive and negative selection process of the immune cells in the thymus, the bone marrow and the lymphatic system. MHC class II molecules bind and present peptides, which are ingested from the immediate cellular environment and processed by a variety of enzymes in vesicles, generated by the fusion of lysosomes and phagosomes. These class II molecules activate CD4+ (T-helper cells) cells, which in turn activate B-cells, thus inducing the humoral immune response.

A peptide is immunogenic if it is capable of binding to MHC molecules and has the ability to be recognized by CD8+ or CD4+ T cells. In this context "immunogenicity" is the ability of an antigen to provoke an immune response. Immunogenicity of an antigen is defined by many variables including size, structure, stability, difference to endogenous molecules, adjuvant presence and the immune condition of the organism as well as other genetic factors.

Only then it can induce a response of the immune system, comprising antibodies that specifically recognize antigenic determinants within said antigenic peptide. When the antigen is no foreign protein or peptide, it is normally not recognized by T cells, since these cells are selected not to react with endogenous proteins, and its presentation in the MHC complex on the surface of an antigen-presenting cell is not sufficient to provoke an immune response.

The use of proteins or glycoproteins in the development of new and effective vaccines is controversial, as, in most cases, it has proven to be either ineffective or hazardous to the inoculated organism. This property is due to a lack of immunogenicity based on a non-favourable size, structure, stability or homology to endogenous proteins, or to the inclusion of non-protective epitopes. The use of synthetic peptides can circumvent many of the problems associated with proteins of recombinant origin. Advantages are the specific selection of clearly defined epitopes, exclusion of infectious proteins or peptides, the well defined structure and sequence, easy production procedures and reasonable production costs. One major disadvantage is the poorly defined three-dimensional solution structure that can prevent proper immunization because the peptide structurally differs significantly from its protein archetype. The peptide is conformationally less constrained than the respective protein and the increased flexibility can cause completely different characteristics. In addition, native peptides have a low systemic stability and are rapidly degraded by proteases Another disadvantage is that it is not guaranteed that the peptide is capable to bind to major histocompatibility complex (MHC) proteins class I or II, which is absolutely necessary for the elicitation of an immune response.

The present invention relates to antigenic peptides comprising Aβ variants or fragments thereof, with chemical properties that predispose them for the use as pharmacologically active agents, further characterized by their cyclic nature.

In nature cyclopeptides occur as natural metabolites in the form of hormones, antibiotics, ion transport systems and toxins. In the living organism they are definitely more resistant against proteolytic degradation compared to their respective linear counterparts. Yet, this feature alone makes them promising candidates for the development of pharmacologically active agents. But in addition to their increased metabolic stability they provide more characteristics that make them superior to their linear homologs. Their conformational rigidity allows to shape secondary structure elements as β-sheets or β- and γ-loops in relative short peptides and even to mimic tertiary structure elements. Thus, the disadvantage of linear peptides based on their poorly defined conformation and their high flexibility can be easily overcome. The reduced conformational flexibility, the property to present certain functionalities in a defined and predictable way and the increased selectivity, makes them highly suitable for the use as synthetic pharmacophors. Besides of said advantages, they are most often characterized by a very good bioavailability.

Cyclopeptides can be subdivided by topology into head-tail-, head-side chain-, tail-side chain- or side chain-side chain-cyclized peptides. Furthermore, one can distinguish between homodet, containing only peptide bonds, or heterodet, in addition containing disulfide-, ester- or thioester-bonds, cyclopeptides. To circumvent the racemisation of C-terminal amino acids during cyclization, Prolin or Glycin residues are preferred at the C-terminus. Components as D-amino acids, Prolins, Glycins and N-alkylated amino acids can be used to accelerate the cyclization process by several orders of magnitude.

The antigenic peptides of the present invention are represented by the following formula: 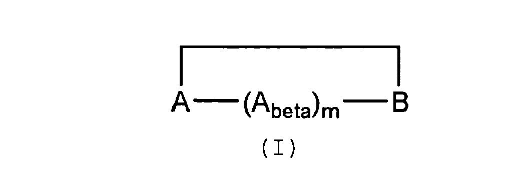 wherein A_{beta} is a peptide of 8-26 amino acid residue length derived from the β-amyloid peptide (Aβ₁₋₂₆; SEQ ID NO: 2) or an analog thereof containing a conservative amino acid substitution;
wherein m is 1 to 6;
wherein A and B represent a direct bond, a linking template or stand for an amino acid sequence comprising 1 to 20 amino acids or derivatives thereof and are selected independently from each other; and
wherein A and B are covalently linked to cyclize the peptide.

In a highly preferred embodiment A_{beta} is a peptide of 12-20 amino acid residue length derived from the β-amyloid peptide (Aβ₁₋₂₆; SEQ ID NO: 2), preferably Aβ₁₋₁₆ (SEQ ID NO: 3) . In another highly preferred embodiment A_{beta} is Aβ₁₁₋₂₆ (SEQ ID NO: 4).

The antigenic peptides of the present invention have preferably a length of 10 to 196 amino acids.

In a certain embodiment, the peptides of the present invention have a length from 10 to 40 amino acid residues, preferably from about 12 to 30 amino acid residues, and more preferably from 14 to 20 amino acid residues.

In a preferred embodiment of the invention m is 1, 2 or 3, more preferably 1.

If A and B represent a direct bond, any two of the amino acids of A_{beta}, preferably the N- and C-terminal amino acid, are covalently linked to cyclize the peptide. The covalent bond can be formed utilizing the N-terminal amino- and the c-terminal carboxy-group, but can also be formed by utilizing any side chain of any of the amino acids of A_{beta}.

If A and B are a linking template, A and B together are a molecule or chemical group that cyclizes A_{beta}. The linking template fixes the Abeta chain in a favorable loop structure and, thus, constrains the flexibility of the antigenic peptide. In a preferred embodiment of the present invention the linking template is a C₅-C₁₄ mono- or polycyclic ring or ring system, that optionally contains one or more heteroatoms selected from the group consisting of N, O and S. In a preferred embodiment said ring system may be substituted with at least two functional groups that allow the covalent bonding of the amino acid chain and are preferably selected from the group consisting of carboxy, hydroxy, amid, amino or imino. In one embodiment said imino group can also be part of said ring or ring system. Said linking template forms, preferably via said functional groups covalent bonds with the N-terminal amino on one end and, on the other end, the C-terminal carboxy group of the amino acid chain of A_{beta}. In another embodiment those substituents covalently bond side chain groups of any of the amino acids of A_{beta}. The C₅-C₁₄ mono- or polycyclic ring or ring system can be further substituted with C₁-C₆ alkyl, C₁-C₆ alkoxy, aryl, aryl-C₁-C₆ alkyl, aroyl-C₁-C₆ alkyl, allyl, halogen, NO₂ or a group of formula CH₂-COOR', wherein R' is hydrogen, C₁-C₆ alkyl, substituted C₁-C₆ alkyl, aryl-C₁-C₆ alkyl, aryl, aroyl-C₁-C₆ alkyl or allyl. If present, the latter groups are preferably substituted by at least two of the above explained functional groups so that said linking forms, via said functional groups, covalent bonds with an amino group and a carboxy group of A_{beta}.

In a highly preferred embodiment the linking template is selected form the group comprising compounds of the following formulae: 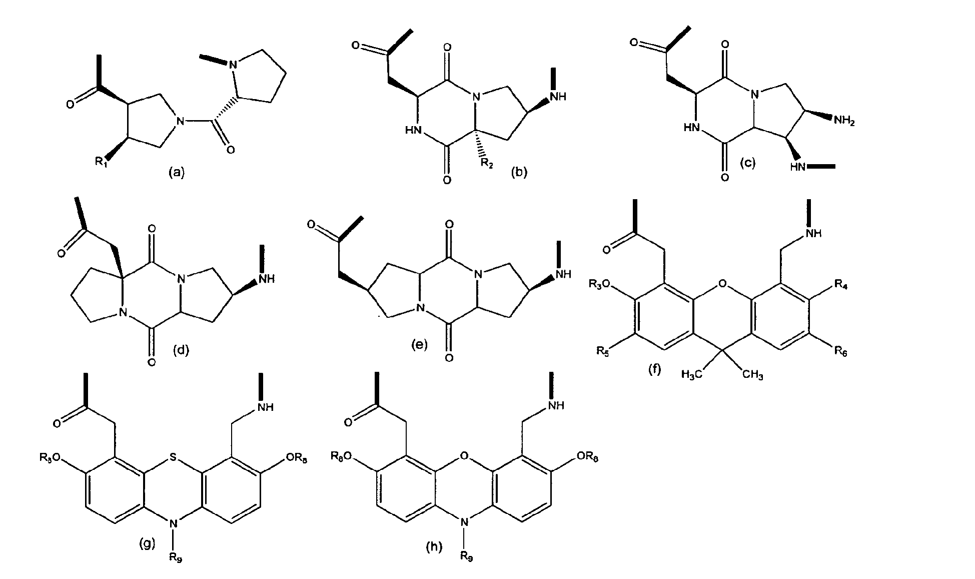 wherein R₁ is hydrogen or an amino group;
R₂ is hydrogen or a group of formula CH₂-COOR₉;
R₃ is hydrogen, C₁-C₆ alkyl or aryl-C₁-C₆ alkyl;
R₄ is hydrogen, C₁-C₆ alkyl, C₁-C₆ alkoxy or aryl-C₁-C₆ alkyl;
R₅ is hydrogen, C₁-C₆ alkyl, substituted C₁-C₆ alkyl, aryl, Br or NO₂;
R₆ is hydrogen, C₁-C₆ alkyl, substituted C₁-C₆ alkyl, aryl, Br or NO₂;
R₇ is hydrogen, C₁-C₆ alkyl, substituted C₁-C₆ alkyl, or aryl-C₁-C₆ alkyl;
R₈ is hydrogen, C₁-C₆ alkyl, substituted C₁-C₆ alkyl, or aryl-C₁-C₆ alkyl; and
R₉ is hydrogen, C₁-C₆ alkyl, substituted C₁-C₆ alkyl, aryl-C₁-C₆ alkyl, aryl, aroyl-C₁-C₆ alkyl or allyl.

If A and B are amino acid residues, then they can be any naturally occurring amino acid or any non-naturally occurring amino acid or derivatives thereof. Non-naturally occurring amino acids include, but are not limited to, hydroxyprolin, aminoprolin, thyroxin, ornithine, norvaline, norleucine, beta-alanine, gamma-amino butyric acid, homoserine, citrulline and the like. Naturally occurring amino acids include glycine, alanine, valine, leucine, isoleucine, methionine, proline, phenylalanine, tyrosine, tryptophan, serine, threonine, cysteine, glutamine, asparagine, histidine, lysine, arginine, glutamic acid and aspartic acid. Also included are explicitly both enantiomers of above-listed amino acids, i.e. the L- and the respective D-form. In this context "derivatives" refers to the above listed amino acid that are modified by additional functional groups, preferably hydroxy, carboxy, amino, C₁-C₆ alkyl, amido, and guanido.

In one preferred embodiment, A is 4-aminoprolin and B is D-Prolin.

The cyclization can be achieved via direct coupling of the N- and C-terminus to form a peptide bond, but can also occur via the amino acid side chains. Furthermore it can be based on the use of other functional groups, including but not limited to amino, hydroxy, sulfhydryl, halogen, sulfonyl, carboxy or thiocarboxy. These groups can be located at the amino acid side chains or be attached to their N- or C-terminus.

In a highly preferred embodiment the antigenic peptide is Cyclo(cis-4-Amino-Pro-Asp-Ala-Glu-Phe-Arg-His-Asp-Ser-Gly-Tyr-Glu-Val-His-His-Gln-Lys-D-Pro).

The amino acids of A and B may be independently from each other modified with lipid moieties. Said lipid moiety is a mostly hydrophobic molecule composed of one or more hydrophobic groups including but not limited to Lipid A, cholesterol, sphingolipids, glycerolbased lipids, diacylglycerol, PEG-lipids, dietherlipids, 1,2-Dioleoyloxy-3-trimethylammonium-propane (DOTAB), 1,2-Dioleoyloxy-3-dimethylammonium-propane (DODAP·Cl), N,N-Dioleoyl-N, N-dimethylammonium(DODAC), fatty acids, isoprenoids, sphingosin and derivatives thereof. The fatty acids include, but are not limited to caproic acid, caprylic acid, capric acid, lauric acid, myristic acid, palmitic acid, stearic acid, arachidic acid, behenic acid, lignoceric acid, myristoleic acid, palmitoleic acid, petroselinic acid, elaidic acid, , oleic acid, linoleic acid, linolelaidic acid, cis-vaccenic acid, linolenic acid, arachidonic acid, cis-11-eicosenoic acid, erucic acid, cis-4,7,10,13,16,19-Docosahexaenoic acid and nervonic acid. The glycerolbased lipids include, but are not limited to phosphatidylethanolamine, phosphatidylserine, phosphatidylcholin (lecithin), phosphatidylinositol, phosphatidylglycerol, cardiolipin, plasmalogen, phosphalic acid and lysophosphatidic acid. The isoprenoids include, but are not limited to farnesyl and geranylgeranyl.

The lipid moiety can be attached directly or via a linker to either or both of the amino acids of A and/or B. Direct linkage is achieved via an amid, ether, thioether, ester or thioester bond. The linker can be any residue, but preferably is chosen from the group of C₃-C₁₀ dicarboxylic acids, and most preferably is succinic acid. The modification can take place at the side chain, or the respective amino or carboxy group of said amino acids. The lipid moiety can be a phospholipid, preferably phosphatidylethanolamine, optionally coupled to succinic acid as a linker. Most preferably the lipid moiety is diacyl-glycero-phosphoethanolamino-succinate, and more preferably 1,3-dipalmitoyl-glycero-2-phosphoethanolamino-succinate.

The lipid modified peptide antigens can, thus, be anchored on the surface of a delivery vehicle, such as liposomes and virosomes.

The invention also relates to immunogenic compositions comprising at least one antigenic peptide represented by formula I as defined above.

The peptide antigens of the present invention can be prepared in a wide variety of ways, known to those skilled in the art. Because of its relatively small size, the peptide can be synthesized in solution or, more sophisticated, on a solid phase, for example a synthetic resin. Various automatic synthesizers are commercially available to date and can be used in accordance with well-investigated synthesis protocols.

Alternatively, the peptides can be expressed recombinantly from synthetic genes in appropriate organisms. These techniques are also readily available for those skilled in the art.

Said methods for preparing the peptides of the invention are also intended to fall into the scope of the present invention as well as the peptides obtained therewith. In a preferred embodiment the peptide is synthesized by employing a method for preparing a peptide of formula I, including the steps of sequentially synthesizing a linear peptide and cyclizing the linear peptide to obtain the peptide of formula I.

Also intended to fall into the scope of the present invention is a peptide obtainable by said method and being represented by the following formula: 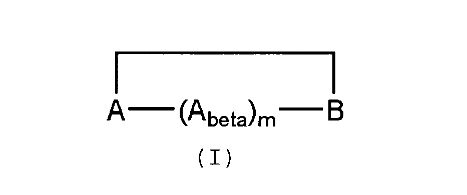 wherein A_{beta} is a peptide of 8-26 amino acid residue length derived from the β-amyloid peptide (Aβ₁₋₂₆; SEQ ID NO: 2) or an analog thereof containing a conservative amino acid substitution;
wherein m is 1 to 6;
wherein A and B represent a direct bond, a linking template or stand for an amino acid sequence comprising 1 to 20 amino acids or derivatives thereof and are selected independently from each other; and
wherein A and B are covalently linked to cyclize the peptide.

The present invention also contemplates the use of the above-described antigenic peptides and immunogenic compositions for the preparation of pharmaceuticals for the diagnosis, treatment and prevention of amyloid-associated diseases. In this context "prevention" refers to the use of said pharmaceutical compositions for the vaccination against said disorders.

### Delivery vehicles

The antigenic peptides can be combined with human compatible delivery vehicles, including but not limited to microparticles (e.g. microspheres and nanospheres), polymeres, bacterial ghosts, bacterial polysaccharides, polypeptides, proteins, attenuated bacteria, virus like particles, attenuated viruses, ISCOMS, liposomes and preferably virosome (IRIVs). The peptides of the invention can attached to or incorporated into the delivery vehicles for the efficient generation of antigenic peptide specific immune responses. "Coupling to" or "attached to" refers to the attachment of the antigenic peptide to the delivery vehicle. This can be done by the means of a covalent linkage or a intermolecular interaction, e.g. hydrogen-bonds or van der Waals forces. In particular the lipid modified peptide antigens can, thus, be anchored on the surface of lipid containing delivery vehicles, such as liposomes and virosomes. "Incorporated into" or "encapsulated in" refers to an antigenic peptide that is within a delivery vehicle, such as microparticles, bacterial ghosts, attenuated bacteria, virus like particles, attenuated viruses, ISCOMs, liposomes and preferably virosomes.

### Virosomes

Additionally the present invention discloses the use of virosomes, capable of eliciting an immune response, by using said virosomes as antigen delivery vehicles for the above-mentioned compounds and inoculating a subject therewith.

Virosomes are envelopes of viruses, and do not contain the infectious genetic material of the original virus. Thus, virosomes are highly effective adjuvants in modern vaccination, possessing superior properties as antigen delivery vehicles and a strong immunogenic potential whilst concomitantly minimizing the risk of side effects. In the present invention compositions are disclosed that comprise beta amyloid antigens incorporated in an immunoadjuvant composition comprising synthetic spherical virosomes termed Immunopotentiating Reconstituted Influenza Virosomes (IRIVs). IRIVs are spherical, unilamellar vesicles with a mean diameter of 150 nm and comprise a double lipid membrane, consisting essentially of phospholipids, preferably Phosphatidylcholines (PC) and Phosphatidylethanolamines (PE). In contrast to liposomes, IRIVs contain the functional viral envelope glycoproteins hemagglutinin (HA) and neuraminidase (NA) intercalated in the phospholipid bilayer membrane. The biologically active HA does not only confer structural stability and homogeneity to virosomal formulations but also significantly contributes to the immunological properties by maintaining the fusion activity of a virus.

To date virosomes have been used effectively in a variety of vaccines. For example, commercially available vaccines against hepatitis A and B, the influenza virus and typhoid fever use virosomes as adjuvants and safe antigen delivery vehicles. Antibodies elicited by the inoculation with antigens reconstituted in virosomes have shown a high affinity for the antigens against which they are raised.

Injected alone the amyloid beta peptide exhibits a relatively low immunogenicity, as would be expected for an endogenous protein. But in a combined form of antigen and virosome, measurable titers of highly specific antibodies against the antigen were produced. Therefore, the present invention also discloses the composition and preparation of amyloid beta peptide antigens bound to virosomes for the diagnosis, treatment or prevention of amyloid-associated disorders. For the production of the described compositions the antigenic peptides are modified with a lipid moiety, and during reconstitution of the virosomes incubated therewith, allowing insertion into the virosome membrane and, thus, noncovalent attachment of the antigenic peptide to the virosomal surface. Alternatively the antigenic peptides can be transported unmodified inside the virosome. The difference lies in the type of immune response. When the virosomes fuse with the target cells, they release their content into the cellular cytoplasm. There that content is processed and presented in complex with MHC class I molecules on the cell surface, triggering the cellular, CD8+ cell-mediated, cytotoxic immune response. In contrast to that mechanism, if the surface antigen is recognized, endocytosed and presented in the MHC class II, the humoral immune response and the production of specific antibodies is elicited. It is believed that the mechanism of virosome action after administration of a composition of the present invention, involves the active binding of the IRIV/antigenic peptide complex to the surface of macrophages, leading to phagocytosis of the virosome/antigen complex or the fusion of virosomal and plasma membrane. Incorporated antigens are processed and the fragments presented on the macrophage surface. This stimulates T-lymphocyte activation, which, in turn, elicits. the production of anti amyloid antibodies by the B-lymphocytes.

The peptide antigens of the present invention can be attached to or enclosed in the virosome. Attachment to or insertion in the virosomal membrane is achieved by modifying the peptide with a lipid moiety as described above. To enclose an antigenic peptide in the virosome, a modification is not necessary.

In one preferred embodiment of the invention the antigen delivery vehicle is a virosome and the antigenic peptide is attached to the virosomal surface via a lipid moiety

The present invention also provides methods for the diagnosis and treatment of amyloid-associated diseases as well as methods for the vaccination against said disorders using above-mentioned compositions. In one embodiment of this invention a immunogenic composition comprising an antigenic peptide of formula I and a virosome is used to facilitate an increase in the level of antibodies specific for amyloid beta peptide, helpful in diagnosis, treatment or prevention of said disorder.

### Adjuvants

Antigens are defined by two characteristic properties: their immunogenicity, meaning their capacity to provoke an immune response in an organism and their antigenicity, that is, their capacity to be recognized by antibodies specific for said antigen. As some antigens are only weakly immunogenic administered by themselves, they fail to induce an immune response necessary for providing effective immunotherapy or protection for an organism. To overcome this deficiency, the use of adjuvants has been demonstrated to be a powerful tool in immunization. An adjuvant functions by enhancing the immune response to a certain antigen, administered in combination with itself. The immunopotentiating mechanism is based on alteration of the pharmacokinetics of the antigen or direct stimulation of the immune system. The use of adjuvants is well known in the art and the skilled person has a variety of suitable adjuvants at its disposal.

The compositions of the present invention also comprise the combination of the invented antigens with adjuvants, known to those skilled in the art, comprising but not limited to oligo- or polysaccharides, Freund's (complete and incomplete), mycobacteria such as BCG, M. Vaccae, or Lipid A, or corynebacterium parvum, quil-saponin mixtures such as QS-21 (SmithKline Beecham), MF59 (Chiron), and various oil/water emulsions (e.g. IDEC-AF). Other adjuvants which may be used include, but are not limited to: mineral salts or mineral gels such as aluminum hydroxide, aluminum phosphate, and calcium phosphate; LPS derivates, saponins, surface active substances such as lysolecithin, pluronic polyols, polyanions, peptides or protein fragments, keyhole limpet hemocyanins, and dinitrophenol; immunostimulatory molecules, such as saponins, muramyl dipeptides and tripeptide derivatives, CpG dinucleotides, CpG oligonucleotides, monophosphoryl Lipid A, and polyphosphazenes; particulate and microparticulate adjuvants, such as emulsions, liposomes, virosomes, cochleates; or immune stimulating complex mucosal adjuvants. Cytokines are also useful in vaccination protocols as a result of lymphocyte stimulatory properties. Many cytokines useful for such purposes will be known to one of ordinary skill in the art, including interleukin-2 (IL-2), IL-12, GM-CSF and many others.

All these adjuvants can be used alone or in combination, creating an adjuvant system. As they can further stimulate immune response, their use in addition to or instead of virosomes is part of the invention.

### Antibodies

The present invention also contemplates antibodies or antigen binding fragments thereof, which bind to the invented compounds and inhibit amyloid deposition and fibril formation. Included in the invention are different types of antibodies, e.g. monoclonal, polyclonal or bispecific antibodies and epitope-recognizing fragments thereof. Furthermore, the antibodies may be humanized or chimeric antibodies, comprising parts of a human antibody as well as parts of antibody regions from other species.

The antibodies of this invention preferably recognize human Abeta proteins or peptides, but also include antibodies directed against amyloid peptides from other species, preferably mammals, and include polyclonal as well as monoclonal antibodies

The invention also relates to a method for the preparation of an antibody that specifically recognizes the antigenic peptides of the invention, comprising the steps of:
(a) immunizing an organism with an immunogenic composition comprising at least one of the antigenic peptides represented by formula I;
(b) isolating the antibodies generated by the inoculation in step (a); and
(c) screening the antibodies obtained in step (b) for their specific recognition of the Aβ peptide fragments or variants thereof.

The invention also relates to an antibody obtainable by the above-described method and its use in the diagnosis, treatment and prevention of amyloid-associated diseases.

### Administration

The present invention also provides for the administration of immunostimulatory compositions, containing the beta amyloid-derived antigens optionally in combination with adjuvants and/ordelivery vehicles in a suitable pharmaceutical formulation.

Such a immunogenic composition which contains one or more of the amyloid-derived antigenic peptides and/or peptide containing compositions of the present invention, as the principal or member active ingredient, can be administered in a wide variety of therapeutic dosage forms in the conventional vehicles for topical, oral, systemic, local, and parenteral administration. Thus, the invention provides compositions for parenteral administration which comprise a solution of the antigenic peptides dissolved or suspended in an acceptable carrier, preferably an aqueous carrier, e.g., water, buffered water, 0.4% saline, 0.3% glycine, hyaluronic acid and the like known to a person skilled in the art. These compositions may be sterilized by conventional, well-known sterilization techniques, or may be sterile filtered. The resulting aqueous solutions may be packaged for use as is, or lyophilized, the lyophilized preparation being combined with a sterile solution prior to administration.

The compositions may optionally contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, among many others known to a person skilled in the art. Actual methods for preparing parenterally administrable compounds will be known or apparent to those skilled in the art and are described in more detail in, for example, Remington: The Science and Practice of Pharmacy ("Remington's Pharmaceutical Sciences") by Gennaro A.R., ed. 20th edition, 2000: Williams & Wilkins PA, USA, which is incorporated herein by reference.

The route and regimen of administration will vary depending upon the stage or severity of the condition to be treated, and is to be determined by the skilled practitioner. For example, the peptides and peptide-containing compositions can be administered in such oral dosage forms as for example tablets, capsules (each including timed release and sustained release formulations), pills, powders, granules, elixirs, tinctures, solutions, suspensions, syrups and emulsions, or alternatively by injection. Similarly, they may also be administered in intravenous (either by bolus or infusion methods), intraperitoneal, subcutaneous, topical with or without occlusion, or intramuscular form. In preferred embodiments, the antigenic peptides and peptide-containing compositions are administered intradermally or subcutaneously. All of these forms are well known to those of ordinary skill in the pharmaceutical arts.

Advantageously, suitable formulations of the present invention may be administered in a single dose, or the total dosage may be administered in divided doses for example of two, three, or four times. Furthermore, compounds of the present invention, particularly those containing virosomes or liposomes, can be administered in intranasal form, or via transdermal routes known to those of ordinary skill in the art.

The antigenic peptides and compositions of the present invention may be administered in a pharmaceutical composition comprising the active compound in combination with a pharmaceutically acceptable carrier adapted for topical administration. Topical pharmaceutical compositions may be, for example, in the form of a solution, cream, ointment, gel, lotion, shampoo, or aerosol formulation adapted for application to the skin. These topical pharmaceutical compositions containing the compounds of the present invention ordinarily include about 0.005% to 5% by weight of the active compound in admixture with a pharmaceutically acceptable vehicle.

The dosage regimen utilizing the compositions of the present invention is selected in accordance with a variety of factors, including for example species, age, weight, sex and medical condition of the patient, the stage and severity of the condition to be treated, and the particular compound thereof employed. A physician of ordinary skill can readily determine and prescribe the effective amount of the drug required to prevent, counter, or arrest the progress of an amyloid-associated disease. Optimal precision in achieving concentration of drug with the range that yields efficacy either without toxicity or with acceptable toxicity requires a regimen based on the kinetics of the drug's availability to target sites. This process involves a consideration of the distribution, equilibrium, and elimination of the drug, and is within the ability of the skilled practitioner.

In the methods of the present invention, the compounds herein described in detail can form the active ingredient and are typically administered in admixture with pharmaceutical diluents or excipients suitably selected with respect to the intended form of administration, that is, oral tablets, capsules, elixirs, syrups, and the like, and consistent with conventional pharmaceutical practices. For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium alginate, carboxymethyl cellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methylcellulose, aga, bentonite, xanthan gum and the like.

The liquid forms may be suitably flavored suspending or dispersing agents such as the synthetic and natural gums, for example, tragacanth, acacia, methyl cellulose and the like. Other dispersing agents, which may be employed, are glycerine and the like.

For parenteral administration, sterile suspensions and solutions are desired. Isotonic preparations that generally contain suitable preservatives are employed when intravenous administration is desired. Topical preparations containing the active drug component can be admixed with a variety of carrier materials well known in the art, such as, for example, alcohols, aloe vera gel, allatoin, glycerine, vitamins A or E oils, mineral oil, PPG2 myristoyl propionate, and the like, to form, for example, alcoholic solutions, topical cleansers, cleansing creams, skin gels, skin lotions, and shampoos in cream or gel formulations.

The antigenic peptides, compositions, or formulation thereof of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates, and cross-linked or amphipathic block copolymers of hydrogels. Generally, subjects can receive an intradermal injection of an effective amount of the antigenic peptides and compositions either in combination with delivery vehicles, such as virosomes, or by themselves. The peptides of the present invention can also be administered in the form of liposome delivery systems, such as small unilamellar vesicles, large unilamellar vesicles and multilamellar vesicles. Liposomes can be formed from a variety of compounds, including for example cholesterol, stearylamine, and various phosphatidylcholines.

Initial doses can be followed by booster doses, following immunization protocols standard in the art. The immunostimulatory effect of the compositions and methods of the present invention can be further increased by combining any of the above-mentioned antigenic peptides, including their combination with virosomes, with a further immune response potentiating compound. Immune response potentiating compounds are classified as either adjuvants or cytokines. Additional adjuvants may further enhance the immunological response by providing a reservoir of antigen (extracellularly or within macrophages), activating macrophages and stimulating specific sets of lymphocytes. Adjuvants of many kinds are well known in the art; specific examples include immunogenic oligonucleotides and peptides, Freund's, alum, mycobacteria such as BCG and M. Vaccae, quil-saponin mixtures such as QS-21 (SmithKline Beecham), and various oil/water emulsions (e.g. IDEC-AF). Also useful in vaccination protocols are cytokines due to their lymphocyte stimulatory properties. Many cytokines useful for such purposes will be known to one of ordinary skill in the art, including interleukin-2 (IL-2), IL-12, GM-CSF and many others.

When administered, the therapeutic compositions of the present invention are administered in pharmaceutically acceptable preparations. Such preparations may routinely contain pharmaceutically acceptable concentrations of salt, buffering agents, preservatives, compatible delivery vehicles, supplementary immune potentiating agents such as adjuvants and cytokines and optionally other therapeutic agents. The preparations of the invention are administered in effective amounts. Generally, doses of immunogens ranging from 1 nanogram/kilogram to 100 milligrams/kilogram, depending upon the mode of administration, are considered effective. The preferred range is believed to be between 500 nanograms and 500 micrograms per kilogram. The absolute amount will depend upon a variety of factors, including the composition selected for administration, whether the administration is in single or multiple doses, and individual patient parameters including age, physical condition, size, weight, and the stage of the disease. These factors are well known to those of ordinary skill in the art and can be addressed with no more than routine experimentation.

In the case of treating an amyloid-associated disease, the desired response is reduction of the amyloid plaques and the systemic concentration of the Aβ peptide.

In the case of prophylaxis, meaning vaccination or immunization, the desired response is protective immunity against the hazardous peptide, as measured by secondary immune responses upon exposure to the amyloid peptide or an antigen thereof.

These desired responses can be monitored by routine methods. The present invention is not to be limited in scope by the specific embodiments described herein. Indeed, various modifications of the invention in addition to those described herein will become apparent to those skilled in the art from the foregoing description, as well as from the examples. Such modifications are intended to fall within the scope of the appended claims.

### Examples

The present invention is illustrated by the following examples, which are not to be construed in any way as imposing limitations upon the scope thereof. On the contrary, it is to be clearly understood that resort may be made to various other embodiments, modifications and equivalents thereof, which after reading the description herein, may suggest themselves to those skilled in the art, but still fall under the scope of the invention.

### Example 1

### Preparation of the virosomes

For the preparation of PE-mimetic-IRIV, a solution of purified Influenza A/Singapore hemagglutinine (4mg) in phosphate buffered saline (PBS) was centrifuged for 30 min at 100 000 g and the pellet was dissolved in PBS (1.33 ml) containing 100 mM octaethyleneglycolmonodecylether (PBS-OEG). Amyloid-peptide-phosphatidylethanolamin conjugates (4mg), phosphatidylcholine (32 mg; Lipoid, Ludwigshafen, Germany) and phosphatidyl-ethanolamine (6 mg) were dissolved in a total volume of 2.66 ml of PBS-OEG. The phospholipids and the hemagglutinine solutions were mixed and sonicated for 1 min. This solution was centrifuged for 1 hour at 100 000 g and the supernatant was sterilized by filtration . Virosomes were formed by detergent removal (SM BioBeads, BioRad, Glattbrugg, Switzerland).

### Example 2

### Vaccination procedure

Double transgenic F1 mice in FVB x C57Bl genetic background (n = 36) were derived by crossing APP [V717I] with PS1 [A246E] transgenic mice (Moechars et al., 1999; Dewachter et al., 2000). All mice were genotyped by PCR at weaning (3 weeks), and re-genotyped at the onset of the study. Mice were randomized for the trials, blinded for the care-takers and experimentators, were age- and sex-matched in the control and treated groups and had free access to water and food. Mice were kept under a reversed day-night cycle with 12 hours light and 12 hours darkness starting at 7 am. All mice were pre-immunized three weeks before the onset of the proper vaccination, at age 5-6 weeks by intra-muscular injection of 100 µl of purified influenza virus (H1/N1 A/Sing, 10 µg/ml in phosphate-buffered saline). This was done to reflect the human situation, since practically everybody tests positive for anti-influenza antibodies. A total of 24 double transgenic mice were vaccinated monthly with 100 µl of Aβ virosomes during 5 months, while 12 control double transgenic mice were treated with control virosomes not expressing amyloid peptide, following an identical scheme.

### Example 3

### Immunohistochemistry and Aβ ELISA of brain tissue

Mice were anaesthetized with a mixture of ketalar (Ketamin), rompun (Xylazin 2%) and atropin (2:1:1). Blood was collected by heart puncture and plasma collected by centrifugation at 14.000 rpm at 4°C for 10 minutes. The mice were flushed trans-cardiacally with ice-cold saline. Brains were removed and left and right hemispheres were processed for biochemical and immunohistochemical analysis. One hemisphere was immediately immersed in liquid nitrogen and stored at -70°C until homogenization for analysis of Aβ peptides by ELISA. The other hemisphere was fixed in 4% paraformaldehyde for immunohistochemistry. All collected samples were labeled with the ID number of the mouse, blind for the annalists and without any reference to the type of treatment.

### Immunohistochemistry

Sagittal vibratome sections (40 µm) were cut for free floating incubations and stored at 4°C until staining. A total of 25 consecutive sections per brain containing the hippocampal region were selected, whereby sections 1, 6, 11, 16, 21 were immuno-stained with Pan β-Amyloid (Ab-1) polyclonal rabbit antiserum (Oncogene, San Diego, CA), sections 2, 7, 12, 17, 22 stained with Thioflavin S and sections 3, 8, 13, 18, 23 with monoclonal antibody CD45 (Pharmingen, San Diego, CA) specific for activated microglia. Sections were randomized and staining and quantitated blindly. Quantitation was performed on images acquired with a Leica DMR microscope equipped with a Sony DXC-9100P camera and analyzed with a computer program (Q-Win software, Leica, Leitz, Germany). Light intensity and condenser settings for the microscope were kept constant throughout the entire image acquisition process. All acquired images were subjected to the same computer subroutines to eliminate investigator bias. Density slice thresholds were applied uniformly throughout analysis. The area of the subiculum was selected for automatic quantification of Aβ immuno-staining and Thioflavin S staining, respectively yielding total amyloid load and dense-cored, senile plaque load. Sections with mechanical or structural artifacts in the subiculum were excluded from analysis.

Pan Aβ immuno-staining was performed using a three step method. Briefly, sections were incubated overnight with Pan Aβ antiserum (1:10 000) diluted in phosphate-buffered saline containing 10% fetal calf serum. Sections were further developed with biotinylated goat anti-rabbit anti-serum (1:500 dilution) and the ABC kit (Vector, Burlingame, CA), using diaminobenzidine and hydrogen peroxide as the substrates. Stained sections were mounted (Depex). Staining for activated microglia was performed similarly, except that anti-CD45 monoclonal antibody (1:5000) and goat anti-rat IgG antiserum (1:500)(Vector, Burlingame, CA) were used.

For thioflavin S staining, the sections were rinsed in tap water, stained for 8 minutes with thioflavin S solution (1% in PBS), fixed in 4% formaldehyde, rinsed and re-stained for 4 minutes. The sections were fixed with 80% ethanol and mounted (Mowiol) with anti-fade (DABCO, 1,4 diazabicyclo (2.2.2) octane), (Sigma, Steinheim, Germany).

### ELISA of soluble and insoluble Aβ

Brains were homogenized in 5 volumes of 20 mM Tris/HCl (pH 8.5) containing a cocktail of protease inhibitors by 10 strokes (650 rpm) in a Potter homogenizer. Homogenates were centrifuged at 4°C for 80 minutes at 135 000 g. From the supernatant the soluble Aβ peptides were isolated by reversed phase column chromatography (C18-Sep-pack Vac 3cc cartridges, Waters, MA). Before sample application, columns were washed with 2 ml of 80% acetonitrile in 0.1% trifluoroacetic acid (TFA) in water and twice with 2 ml of 0.1% TFA in water. Samples were applied and the columns washed with 1.5 ml of 5% acetonitrile/0.1% TFA, 1.5 ml 25 % acetonitrile/0.1 % TFA. The Aβ peptides were eluted with 50 % acetonitrile/0.1% TFA, collected in one fraction that was freeze-dried overnight. The freeze-dried pellet was resuspended in Tris-buffered saline containing protease inhibitors, 2% NP40 and 2% Triton X-100 and subsequently centrifuged for 60 minutes at 98 000 g at 4°C.

Insoluble amyloid peptides are contained in the pellets of the first centrifugation step after homogenization. The pellets were resuspended in 80 mM Tris/HCl pH 8.0 containing 8 M guanidinium chloride by mixing for 3 hours at room temperature and subsequently appropriately diluted in ELISA sample buffer.

Analysis by ELISA of the Aβ40 and Aβ42 peptides in the soluble and insoluble fractions was carried out by dedicated specific assay kits, according to the instructions of the manufacturer (The Genetics Company, Zurich, Switzerland).

## Claims

1. An antigenic peptide represented by the following formula: 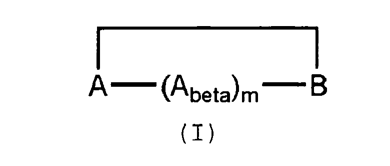 wherein A_{beta} is a peptide of 8-26 amino acid residue length derived from the β-amyloid peptide (Aβ₁₋₂₆; SEQ ID NO: 2) or an analog thereof containing a conservative amino acid substitution;
wherein m is 1 to 6;
wherein A and B are covalently linked to cyclize the peptide;
and wherein A and B represent a direct bond, a linking template or stand for an amino acid sequence comprising 1 to 20 amino acids or derivatives thereof and are selected independently from each other.

2. The peptide of claim 1, wherein A_{beta} is a peptide of 12-20 amino acid residue length derived from the β-amyloid peptide (Aβ₁₋₂₆; SEQ ID NO: 2), preferably Aβ₁₋₁₆ (SEQ ID NO: 3) or Aβ₁₁₋₂₆ (SEQ ID NO: 4).

3. The peptide of claim 1 or 2, wherein A and B represent a direct bond of any two of the amino acids of A_{beta},
wherein the amino acids are covalently linked to cyclize the peptide.

4. The peptide of claim 3, wherein A and B represent a direct bond and the N- and C-terminal amino acid of A_{beta} are covalently linked to cyclize the peptide.

5. The peptide of claim 1 or 2, wherein A and B together represent a linking template and wherein the linking template is a C₅-C₁₄ mono- or polycyclic ring or ring system which may contain one or more hetero atoms.

6. The peptide of claim 5, wherein the linking template is an N-containing heterocyclic C₅-C₁₄ mono- or polycyclic ring or ring system.

7. The peptide of claim 1 or 2, wherein A and B are independently chosen from the group, comprising hydroxyprolin, aminoprolin, thyroxin, ornithine, norvaline, norleucine, beta-alanine, gamma-amino butyric acid, homoserine, citrulline, glycine, alanine, valine, leucine, isoleucine, methionine, proline, phenylalanine, tyrosine, tryptophan, serine, threonine, cysteine, glutamine, asparagine, histidine, lysine, arginine, glutamic acid and aspartic acid wherein the L- or D-forms of each amino acid are comprised.

8. The peptide of claim 7, wherein A is 4-aminoproline.

9. The peptide of claim 7 or 8, wherein B is D-proline.

10. The peptide of claim 1, 2 or 7 to 9, wherein the antigenic peptide of formula I is Cyclo(cis-4-Amino-Pro-Asp-Ala-Glu-Phe-Arg-His-Asp-Ser-Gly-Tyr-Glu-Val-His-His-Gln-Lys-D-Pro).

11. The peptide of any of claims 1-10, wherein the antigenic peptide of formula I, preferably at least one of A and B, is further modified with a lipid.

12. The peptide of claim 11, wherein the lipid is a phospholipid, preferably attached to A or B via a linker molecule.

13. The peptide of claim 12, wherein the linker is a dicarboxylic acid having 3 to 10 carbon atoms, preferably succinic acid.

14. The peptide of any of claims 11 to 13, wherein the lipid is phosphatidylethanolamine.

15. The peptide of claim 14, wherein the lipid is 1,3-dipalmitoyl-glycero-2-phosphoethanolamine and the linker molecule is succinic acid.

16. The peptide of claim 15, wherein the antigenic peptide is Cyclo(cis-4-Amino-Pro(succinyl-(1,3-dipalmitoyl-glycero-2-phosphoethanolamino))-Asp-Ala-Glu-Phe-Arg-His-Asp-Ser-Gly-Tyr-Glu-Val-His-His-Gln-Lys-D-Pro).

17. The use of at least one antigenic peptide of any of claims 1-16 for preparing a pharmaceutical composition for the vaccination of an individual against amyloid-associated diseases.

18. The use of at least one antigenic peptide of any of claims 1-16 for preparing a pharmaceutical composition for the treatment of an individual afflicted with an amyloid-associated diseases.

19. The use of at least one antigenic peptide of any of claims 1-16 for preparing a pharmaceutical composition for the diagnosis of an amyloid-associated disease.

20. An immunogenic composition comprising at least one antigenic peptide represented by formula I as defined in any of claims 1-16.

21. The composition of claim 20, further comprising an antigen delivery vehicle selected from the group consisting of microparticles including microspheres and nanospheres, polymeres, bacterial ghosts, bacterial polysaccharides, polypeptides, proteins, attenuated bacterias, virus like particles, attenuated viruses, ISCOMs, liposomes or virosomes (IRIVs).

22. The composition of 21, wherein the antigen is attached to or incorporated into the delivery vehicle.

23. The composition of claim 21, wherein the antigen delivery vehicle is a virosome, liposome, virus like particle, attenuated virus or ISCOM and the antigenic peptide is attached to the surface via a lipid moiety.

24. The composition of any of claims 20 to 23 further comprising an adjuvant or an adjuvant system.

25. A method for preparing a peptide of formula I, including the steps of:
(a) sequentially synthesizing a linear peptide; and
(b) cyclizing the linear peptide to obtain the peptide of formula I.

26. The method of claim 25, further comprising the step of modifying the peptide of formula I with a lipid moiety.

27. A method for preparing a antibody against the immunogenic peptides represented by formula I, comprising the steps of:
(a) immunizing an organism with an immunogenic composition comprising at least one of the antigenic peptides of formula I;
(b) isolating the antibodies generated by the inoculation in step (a); and
(c) screening the antibodies obtained in step (b) for their specific recognition of the Aβ peptide fragments or variants thereof.

28. An antibody obtained by the method of claim 27.

29. The use of an antibody according to claim 28 for the preparation of a pharmaceutical for the diagnosis, treatment or prevention of an amyloid-associated disease.


Sequence listing

<110>  Pevion Biotech

<120>  Amyloid beta-peptide vaccine

<130>  P201039

<160>  4

<170>  PatentIn version 3.3

<210>  1
<211>  42
<212>  PRT
<213>  Homo sapiens beta-amyloid peptide 1-42

<400>  1

<210>  2
<211>  26
<212>  PRT
<213>  Homo sapiens beta-amyloid peptide 1-26

<400>  2

Asp Ala Glu Phe Arg His Asp Ser Gly Tyr Glu Val His His Gln Lys
1               5                   10                  15

Leu Val Phe Phe Ala Glu Asp Val Gly Ser
            20                  25

<210>  3
<211>  16
<212>  PRT
<213>  Homo sapiens beta-amyloid peptide 1-16

<400>  3

Asp Ala Glu Phe Arg His Asp Ser Gly Tyr Glu Val His His Gln Lys
1               5                   10                  15

<210>  4
<211>  15

<212>  PRT
<213>  Homo sapiens beta-amyloid peptide 11-26

<400>  4

Val His His Gln Lys Leu Val Phe Phe Ala Glu Asp Val Gly Ser
1               5                   10                  15